# EUROPEAN PATENT SPECIFICATION

(11) **EP 3 934 718 B1**
(45) Date of publication and mention of the grant of the patent: **18.11.2026**
(21) Application number: 20720553.5
(22) Date of filing: 04.03.2020
(51) Int. Cl.: A61M 5/162, A61M 5/14

(54) **IV SET SPIKE WITH ENHANCED REMOVAL FORCE**
SPIKE EINES IV-SETS MIT ERHÖHTER ENTFERNUNGSKRAFT
ENSEMBLE POINTE IV À FORCE DE RETRAIT AMÉLIORÉE

(30) Priority: 04.03.2019 US 201916292122
(43) Date of publication of application: 12.01.2022
(73) Proprietor: CareFusion 303, Inc., San Diego, CA 92130 (US)
(72) Inventor: MASON, Gene, La Habra, CA 90631 (US); MANSOUR, George, Diamond Bar, CA 91765 (US); CALLAHAN, Ryan, Long Beach, CA 90814 (US); YU, Edmond, Chino Hills, CA 91709 (US)
(74) Representative: Epping - Hermann - Fischer
(86) International application number: PCT/US2020/021007
(87) International publication number: WO 2020/180996

(56) References cited:
- EP-A1- 0 050 459
- EP-B1- 0 050 459
- WO-A1-2007/047200
- WO-A1-2011/118411
- WO-A1-2012/103141
- US-A1- 2011 077 621

## Description

### FIELD OF THE INVENTION

The present disclosure generally relates to IV spikes, and, in particular, to IV spikes with engagement features.

### BACKGROUND

Medical treatments often include the infusion of a medical fluid (e.g., a saline solution or a liquid medication) to patients using an intravenous (IV) catheter that is connected though an arrangement of flexible tubing and fittings, commonly referred to as an "IV set," to a source of fluid, for example, an IV container, bag, or bottle. An IV set can include an IV spike to pierce a membrane of an IV container to allow the medical fluid to exit the IV container. Once an IV spike is advanced through the membrane of the IV container, medical fluid can flow through the IV spike to the IV set.

In some applications, the IV container can be a blow molded polyethylene bottle. The blow molded bottles may have a thin wall thickness and low compliance. In certain applications, during the use of IV sets, the IV spike may be inadvertently removed from the IV container.

EP 0 050 459 A1 discloses a spike connector (10) of the type in which a hollowed spike (14) extending from the main body portion (12) of the spike connector is inserted into the stopper of a fluid source in order to convey the fluid from the source through the spike (14) and main body portion (12). The main body portion (12) includes a pair of wings (32,34) which extend upwardly in the direction of the spike and outwardly therefrom, with the tops of the wings serving to space the remaining top of the main body portion (12) away from the fluid source stopper to prevent collection of the solution on the top of the main body portion. The undersides (36,38) of the wings (32, 34) form a convenient finger grip. The tip portion (30) of the spike is relatively polished to enable easy insertion of the spike into the stopper.
WO 2007/047200 A1 discloses a pharmaceutical delivery apparatus including a housing (22) , a syringe assembly, and a needle (44) cap. The syringe assembly is plungeable relative to the housing from a first position, at which its needle tip is disposed within the housing, to a second position, at which its needle tip projects from the housing beyond the proximal end for insertion into an injection site. A base (46) of the needle cap is exposed at the housing proximal end to be manually grippable for cap removal. A needle cap stem is upstanding from the base and sized and configured to insert through an opening is the housing proximal end to cover the needle tip when the syringe assembly is disposed in the first position. The needle cap base further includes a plurality of distally projecting cams (54, 258) located radially outward of the stem. The cams are fittable within slots (40, 240) in the housing proximal end when the cap is fully mounted to the apparatus.

### SUMMARY

The invention is defined by the claims. In the following, parts of the description and drawings referring to embodiments which are not covered by the claims are not presented as embodiments of the invention, but as examples useful for understanding the invention.

The disclosed subject matter relates to IV spikes with engagement features. In certain embodiments an IV spike comprises a spike body comprising a first material and a spike portion converging to a point; at least one spike flow port formed through the spike body; a lower flow port in fluid communication with the at least one spike flow port; and an overmolded engagement feature disposed around the spike body, wherein the overmolded engagement feature comprises a second material and is configured to retain the IV spike within an IV container.

In certain embodiments, an IV spike comprises a spike body comprising a first material and a spike portion converging to a point; at least one spike flow port formed through the spike body; a lower flow port in fluid communication with the at least one spike flow port; and an engagement feature extending radially away from the spike body wherein the engagement feature is configured to retain the IV spike within an IV container.

In certain embodiments, a method to transfer fluid from an IV container to a drip chamber comprises engaging an engagement feature of an IV spike against a membrane of the IV container; and directing flow from the IV container to the drip chamber through the IV spike.

It is understood that various configurations of the subject technology will become readily apparent to those skilled in the art from the disclosure, wherein various configurations of the subject technology are shown and described by way of illustration. As will be realized, the subject technology is capable of other and different configurations and its several details are capable of modification in various other respects, all without departing from the scope of the subject technology. Accordingly, the summary, drawings and detailed description are to be regarded as illustrative in nature and not as restrictive.

### BRIEF DESCRIPTION OF THE DRAWINGS

The accompanying drawings, which are included to provide further understanding and are incorporated in and constitute a part of this specification, illustrate disclosed embodiments and together with the description serve to explain the principles of the disclosed embodiments. In the drawings:
FIG. 1 is an elevation view of an IV set, in accordance with various aspects of the present disclosure.
FIG. 2 is an elevation view of an IV spike for use with the IV set of FIG. 1, in accordance with various aspects of the present disclosure.
FIG. 3 is a top view of the IV spike of FIG. 2, in accordance with various aspects of the present disclosure.
FIG. 4 is an elevation view of the IV spike of FIG. 2, in accordance with various aspects of the present disclosure.
FIG. 5 is a perspective view of an IV spike for use with the IV set of FIG. 1, in accordance with various aspects of the present disclosure.
FIG. 6 is a perspective view of an IV spike for use with the IV set of FIG. 1, in accordance with various aspects of the present disclosure.
FIG. 7 is a perspective view of an IV spike for use with the IV set of FIG. 1, in accordance with various aspects of the present disclosure.

### DETAILED DESCRIPTION

The disclosed IV spike incorporates features to engage and seal against various types of IV containers. The IV spike can engage with portions of IV containers to retain the IV spike within the IV container. The detailed description set forth below is intended as a description of various configurations of the subject technology and is not intended to represent the only configurations in which the subject technology may be practiced. The detailed description includes specific details for the purpose of providing a thorough understanding of the subject technology. However, it will be apparent to those skilled in the art that the subject technology may be practiced without these specific details. In some instances, well-known structures and components are shown in block diagram form in order to avoid obscuring the concepts of the subject technology. Like components are labeled with identical element numbers for ease of understanding. Reference numbers may have letter suffixes appended to indicate separate instances of a common element while being referred to generically by the same number without a suffix letter.

The following description is directed to the administration of medical fluid to a patient by a medical practitioner using the disclosed IV spike. Various aspects of the disclosed IV spike may be used in any application where it is desirable to prevent inadvertent removal of an IV spike.

The disclosed IV spike overcomes several challenges discovered with respect to the operation and setup of certain IV sets. One challenge with certain conventional IV spikes is that they may have a low removal force (less than one pound of force) from IV containers of varying or different construction. Because certain conventional IV spikes may be removed with low amounts of force, certain conventional IV spikes may be inadvertently or unintentionally removed from the IV container, interrupting the medical treatment.

Therefore, in accordance with the present disclosure, it is advantageous to provide an IV spike as described herein that can be securely engaged within different IV containers. The disclosed IV spike provides engagement features to engage with various IV containers. Advantageously, the disclosed IV spike allows for clinicians to consistently and reliably utilize the IV spike without inadvertently removing the IV spike from the IV containers. Further, the disclosed IV spike can be reliably used with a variety of IV containers, including polyvinyl chloride (PVC) IV bags and/or blow molded polyethylene infusion bottles. Additionally, the IV spike can seal against IV containers to prevent leaks.

An example of an IV spike that can engage with various IV containers is now described.

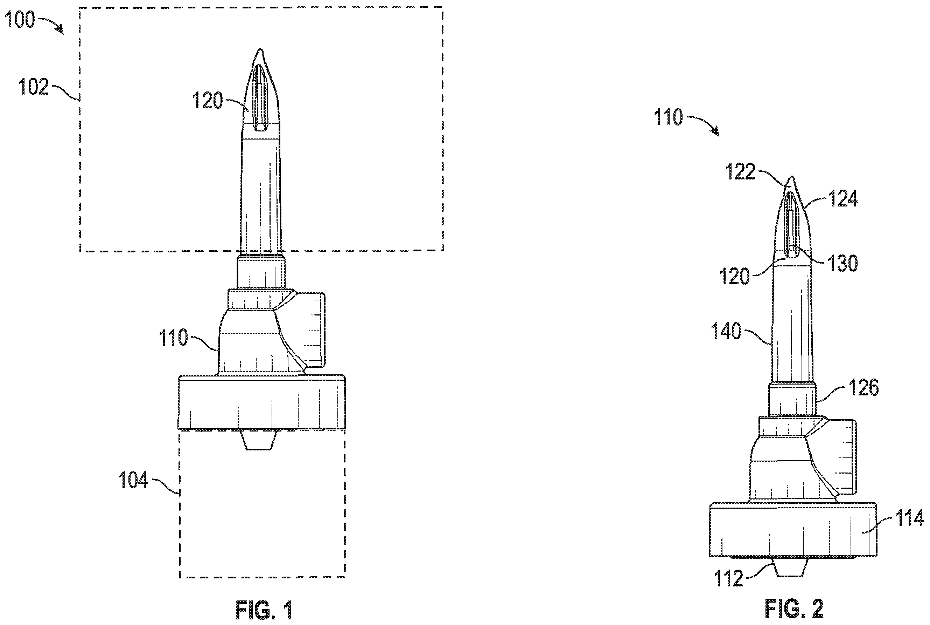

FIG. 1 is an elevation view of an IV set 1 00, in accordance with various aspects of the present disclosure. In the depicted example, the IV set 100 can provide medical fluid from an IV container, bag, or bottle 102 to a patient.

As illustrated, an IV spike 110 facilitates the transfer of fluid from the IV container 102 to the patient via the drip chamber 104. During operation, the IV spike 110 is advanced through the IV container 102 to pierce the membrane of the sealed IV container 102. As the IV spike 110 is advanced, the membrane of the IV container 102 is displaced. After the IV spike 110 is introduced, engagement features of the IV spike 110 retain the IV spike 110 within the IV container 102. Further, the engagement features of the IV spike 110 can seal the IV spike 110 against the IV container 102.

As illustrated, the IV container 102 can be any suitable container to store medical fluid. In some embodiments, the IV container 102 is a PVC IV bag. In some applications, such as some developing countries, the IV container 102 can vary in construction from a PVC IV bag. For example, the IV container 102 can be a blow molded polyethylene infusion bottle. Additionally, the IV container 102 can have thinner wall construction or lower compliance compared to a PVC IV bag.

Advantageously, engagement features of the IV spike 110 can engage with IV containers 102 that have thinner wall construction or lower compliance compared a PVC IV bag to ensure that the IV spike 110 is securely engaged. Optionally, the IV spike 110 can be used with a PVC IV bag.

After the IV spike 110 is introduced into the IV container 102, medical fluid from the IV container 102 is permitted to flow through the IV spike 110 to the drip chamber 104 and to the patient.

FIG. 2 is an elevation view of an IV spike 110 for use with the IV set 100 of FIG. 1, in accordance with various aspects of the present disclosure. In the illustrated embodiment, the IV spike 110 can be advanced through a membrane of an IV container to permit the outflow of the medical fluid within the IV container.

In the depicted example, the spike body 120 pierces and displaces the membrane of the IV container. The spike body 120 extends from a base portion 126 and converges to a spike portion 122. In some embodiments, the spike body 120 is generally an elongate member. Optionally, the spike body 120 can have a generally cylindrical shape. The spike body 120 can be formed from a generally rigid material, including, but not limited to acrylonitrile butadiene styrene (ABS) plastic.

As illustrated, the spike portion 122 converges to a point or more generally to a portion of reduced radius relative to the spike body 120. Optionally, the spike portion 122 can have a bevel 124. During operation, as the IV spike 110 is advanced, the spike portion 122 can pierce or rupture the membrane of the IV container, permitting the spike body 120 to displace the membrane and be advanced into the IV container.

Once the spike body 120 has ruptured the membrane and is advanced into the IV container, the overmold 140 can retain the IV spike 110 within the IV container. For example, the overmold 140 can engage with the edges of the membrane or the IV container surrounding the IV spike 110.

Upon insertion of the spike body 120 into the IV container, the overmold 140 can be sufficiently compliant to elastically deform to compress through the puncture site and/or expand past the puncture site. As a result, the overmold 140 can have a greater surface area in contact with the membrane of the IV container. In some embodiments, the overmold 140 can expand to seal and/or minimize small gaps between the IV spike 110 and the membrane of the IV container to prevent or abate leaks, effectively acting as a gasket. Optionally, edges of the membrane of the IV container can engage or "dig in" to the compliant material of the overmold 140.

In the depicted example, the overmold 140 can be formed of a compliant material, such as a soft polymer, a thermoplastic elastomer (TPE) and/or silicone. In some embodiments, the overmold 140 can be formed from a material with a greater compliance (lower durometer) than the material of the spike body 120.

Furthermore, the overmold 140 can have a sufficient coefficient of friction to frictionally engage the overmold 140 against the membrane of the IV container. In some embodiments, the coefficient of friction of the material of the overmold 140 is greater than the coefficient of friction of the material of the spike body 120.

As illustrated, the overmold 140 is cylindrically and/or circumferentially formed over the spike body 120. In some embodiments, the overmold 140 is formed as a cylindrical body or as a skin over the spike body 120. Optionally, the overmold 140 can extend along an axial length of the spike body 120. The overmold 140 can be formed by a "second shot" during the molding process of the IV spike 110.

Optionally, the overmold 140 can create a physical barrier that prevents the removal of the IV spike 110 from the IV container. For example, edges of the overmold 140 can engage the membrane of the IV container to prevent the removal of the IV spike 110 from the IV container.

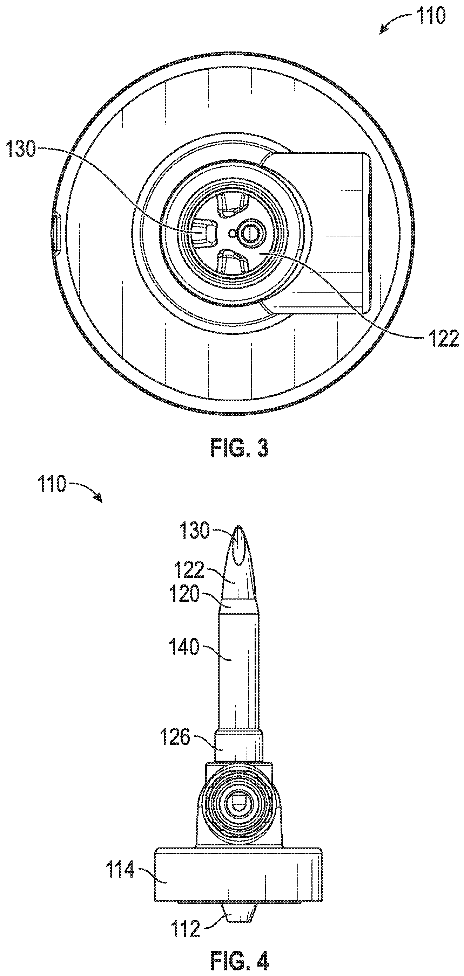

FIG. 3 is a top view of the IV spike 110 of FIG. 2, in accordance with various aspects of the present disclosure. FIG. 4 is an elevation view of the IV spike 110 of FIG. 2, in accordance with various aspects of the present disclosure. With reference to FIGS. 2-4, after the IV spike 110 is introduced into the IV container, the IV spike 110 can facilitate the flow of medical fluid from the IV spike to the patient via the drip chamber.

In the depicted example, medical fluid flows from the spike flow ports 130, through the spike body 120 and exits the IV spike 110 via the lower flow port 112. As illustrated, the spike flow ports 130 can be formed adjacent to or through the spike portion 122 of the spike body 120. Optionally, the spike flow ports 130 can have an oblong cross-sectional profile. In some embodiments, the spike flow ports 130 are disposed above the overmold 140 to permit the flow of medical fluid and air through the spike flow ports 130. In the depicted example, the spike flow ports 130 are formed to continue through the spike body 120. The IV spike 110 can include one or more spike flow ports 130. For example, the IV spike 110 can include three spike flow ports 130.

As illustrated, the spike flow ports 130 are in fluid communication with the lower flow port 112. The lower flow port 112 allows fluid flow from the IV spike 110 to a drip chamber. The drip chamber can be coupled to the IV spike via the drip chamber connector 114.

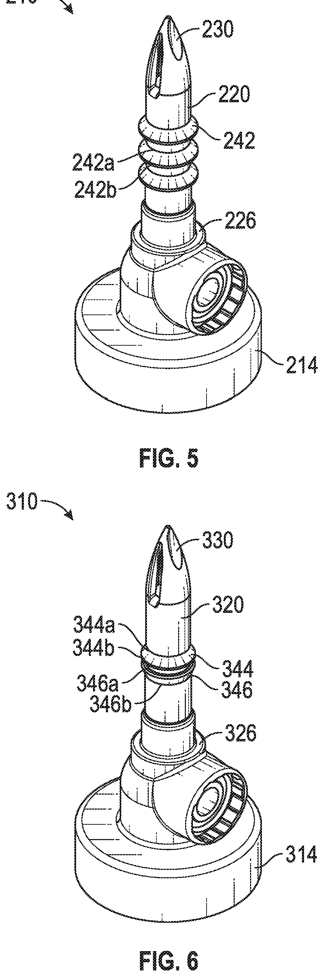

FIG. 5 is a perspective view of an IV spike 210 for use with the IV set 100 of FIG. 1, in accordance with various aspects of the present disclosure. In the depicted example, the IV spike 210 includes retention features 242 that create a physical barrier that prevent the removal of the IV spike 210 from the IV container.

In some embodiments, the retention features 242 extend radially away from the spike body 220. In the depicted example, the retention features 242 have a tapered or barbed shape that allows the retention features 242 to pass through the puncture site. As illustrated, the retention features 242 can be axially spaced apart. In the depicted example, each retention feature 242 includes a distal radius 242a and a proximal radius 242b. The retention feature 242 can be tapered between the distal radius 242a and the proximal radius 242b. As illustrated, the distal radius 242a is smaller than the proximal radius 242b.

Upon insertion of the IV spike 210, the proximal radius 242b of the retention feature 242 can engage against the membrane of the IV container. Advantageously, the proximal radius 242b of the retention feature 242 can provide a greater surface area to engage the membrane of the IV container to prevent the removal of the IV spike 210 from the IV container. In some applications, the retention features 242 can enlarge the puncture site as the IV spike 210 is introduced.

In the depicted example, the retention features 242 can be overmolded or formed of a compliant material, such as a soft polymer, a thermoplastic elastomer (TPE) and/or silicone. In some embodiments, the retention features 242 can be formed from a material with a greater compliance (lower durometer) than the material of the spike body 220.

Optionally, the retention features 242 can be formed from a same or similar material as the spike body 220. In some embodiments, the retention features 242 are integrally formed with the spike body 220.

FIG. 6 is a perspective view of an IV spike 310 for use with the IV set 100 of FIG. 1, in accordance with various aspects of the present disclosure. In the depicted example, the IV spike 310 includes an upper retention feature, barb, or chamfer face 344 and a lower retention feature, barb, or chamfer face 346 that cooperatively create a physical barrier that prevent the removal of the IV spike 310 from the IV container.

In some embodiments, the upper retention feature 344 and the lower retention feature 346 extend radially away from the spike body 320. In the depicted example, the upper retention feature 344 and the lower retention feature 346 each have a tapered shape. As illustrated, the upper retention feature 344 and the lower retention feature 346 can be axially spaced apart.

In the depicted example, the upper retention feature 344 includes an upper distal radius 344a and an upper proximal radius 344b. The upper retention feature 344 can be tapered between the upper distal radius 344a and the upper proximal radius 344b. As illustrated, the upper distal radius 344a is smaller than the upper proximal radius 344b. In some embodiments, the smaller upper distal radius 344a of the upper retention feature 344 allows the upper retention feature 344 to pass through the puncture site.

Similarly, the lower retention feature 346 includes a lower distal radius 346a and a lower proximal radius 346b. The lower retention feature 346 can be tapered between the lower distal radius 346a and the lower proximal radius 346b. As illustrated, the lower distal radius 346a is larger than the lower proximal radius 346b.

Upon insertion of the IV spike 310, the upper proximal radius 344b of the upper retention feature 344 can engage against the membrane of the IV container. Similarly, the lower distal radius 346a of the lower retention feature 346 can engage against the opposite side of the membrane of the IV container, allowing the membrane to be retained (creating a "hard stop") between the upper retention feature 344 and the lower retention feature 346 to prevent the removal of the IV spike 310 from the IV container. In some embodiments, the use of the upper retention feature 344 and the lower retention feature 346 permits for the reuse of an IV container with the IV spike 310.

In the depicted example, the upper retention feature 344 and the lower retention feature 346 can be overmolded or formed of a compliant material, such as a soft polymer, a thermoplastic elastomer (TPE) and/or silicone. In some embodiments, the upper retention feature 344 and the lower retention feature 346 can be formed from a material with a greater compliance (lower durometer) than the material of the spike body 320.

Optionally, the upper retention feature 344 and the lower retention feature 346 can be formed from a same or similar material as the spike body 320. In some embodiments, the upper retention feature 344 and the lower retention feature 346 are integrally formed with the spike body 320.

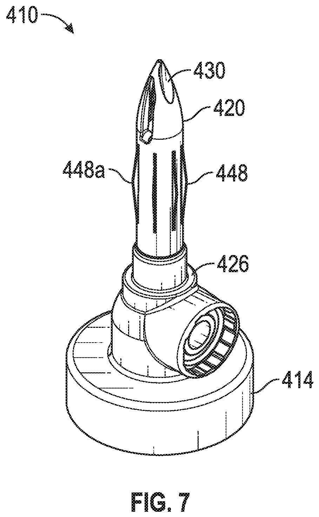

FIG. 7 is a perspective view of an IV spike 410 for use with the IV set 100 of FIG. 1, in accordance with various aspects of the present disclosure. In the depicted example, the IV spike 210 includes axial retention features 448 that create a physical barrier that prevent the removal of the IV spike 410 from the IV container.

In some embodiments, the axial retention features 448 extend axially along and radially away from the spike body 420. In the depicted example, the axial retention features 448 have a ribbed shape that smoothly tapers to a central radial height 448a that allows the axial retention features 448 to pass through the puncture site. In some embodiments, the axial retention features 448 can be radially spaced apart.

Upon insertion of the IV spike 410, the taper of the axial retention features 448 can engage against the membrane of the IV container. Advantageously, axial retention features 448 can provide a greater surface area to engage the membrane of the IV container to prevent the removal of the IV spike 410 from the IV container.

In the depicted example, the axial retention features 448 can be overmolded or formed of a compliant material, such as a soft polymer, a thermoplastic elastomer (TPE) and/or silicone. In some embodiments, the axial retention features 448 can be formed from a material with a greater compliance (lower durometer) than the material of the spike body 420.

Optionally, the axial retention features 448 can be formed from a same or similar material as the spike body 420. In some embodiments, the axial retention features 448 are integrally formed with the spike body 420.

In some embodiments, the IV spikes described herein can include one or more or any combination of the retention features described herein. Additionally, and without limitation, additional retention features can include O-rings, toroidal engagement features, spherical protrusions or engagement features, and/or spikes. Optionally, an IV spike can include retention features that are manipulated by the clinician or by introducing the IV spike to the IV container. For example, an IV spike can include a movable collar, a rotating cam assembly, or a movable oblong retention feature. In some embodiments, an IV spike can include retention features that are expandable after inserting the IV spike into the IV container. For example, an IV spike can include an expandable spike body, and/or a fluid absorbable retention feature. In some embodiments, an IV spike can include external retention features, including, but not limited to outer retainers that engage an outer surface of an IV container and/or hooks that engage an outer surface of an IV container.

The present disclosure is provided to enable any person skilled in the art to practice the various aspects described herein. The disclosure provides various examples of the subject technology, and the subject technology is not limited to these examples. Various modifications to these aspects will be readily apparent to those skilled in the art, and the generic principles defined herein may be applied to other aspects.

A reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention.

The word "exemplary" is used herein to mean "serving as an example or illustration." Any aspect or design described herein as "exemplary" is not necessarily to be construed as preferred or advantageous over other aspects or designs. In one aspect, various alternative configurations and operations described herein may be considered to be at least equivalent.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "embodiment" does not imply that such embodiment is essential to the subject technology or that such embodiment applies to all configurations of the subject technology. A disclosure relating to an embodiment may apply to all embodiments, or one or more embodiments. An embodiment may provide one or more examples. A phrase such an embodiment may refer to one or more embodiments and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such a configuration may refer to one or more configurations and vice versa.

In one aspect, the term "coupled" or the like may refer to being directly coupled. In another aspect, the term "coupled" or the like may refer to being indirectly coupled.

Terms such as "top," "bottom," "front," "rear" and the like if used in this disclosure should be understood as referring to an arbitrary frame of reference, rather than to the ordinary gravitational frame of reference. Thus, a top surface, a bottom surface, a front surface, and a rear surface may extend upwardly, downwardly, diagonally, or horizontally in a gravitational frame of reference.

Various items may be arranged differently (e.g., arranged in a different order, or partitioned in a different way) all without departing from the scope of the subject technology. Furthermore, to the extent that the term "include," "have," or the like is used, such term is intended to be inclusive in a manner similar to the term "comprise" as "comprise" is interpreted when employed as a transitional word in a claim.

The Title, Background, Summary and Brief Description of the Drawings are hereby incorporated into the disclosure and are provided as illustrative examples of the disclosure, not as restrictive descriptions. In addition, in the Detailed Description, it can be seen that the description provides illustrative examples and the various features are grouped together in various embodiments for the purpose of streamlining the disclosure.

The scope of the invention is defined by the appended set of claims.

## Claims

1. An IV spike (310), comprising:
a spike body comprising a first material and a spike portion converging to a point;
at least one spike flow port formed through the spike body;
a lower flow port in fluid communication with the at least one spike flow port;
and **characterized by**:
an engagement feature overmolded from a second material and extending radially away from the spike body wherein the engagement feature is configured to retain the IV spike (310) within an IV container, the engagement feature comprising:
an upper axial retention feature (344) with an upper axially distal radius (344a) and an upper axially proximal radius (344b), wherein the upper axial retention feature (344) tapers from the smaller axially distal radius to the larger upper axially proximal radius (344b); and
a lower axial retention feature (346) with a lower axially distal radius (346a) and a lower axially proximal radius (346b), wherein the lower axial retention feature (346) tapers from the larger axially distal radius to the smaller lower axially proximal radius (346b).

2. The IV spike (310) of Claim 1, wherein the second material is a compliant material.

3. The IV spike (310) of Claim 1, wherein the first material comprises acrylonitrile butadiene styrene plastic.

4. The IV spike (310) of Claim 1, wherein the second material comprises a greater coefficient of friction than the first material.

5. The IV spike (310) of Claim 1, wherein the second material comprises a thermoplastic elastomer or silicone.

6. The IV spike (310) of Claim 1, wherein the engagement feature extends along an axial length of the spike body.

7. The IV spike (310) of Claim 1, wherein the engagement feature comprises a cylindrical body.

8. The IV spike (310) of Claim 7, wherein the engagement feature is disposed circumferentially around the spike body.

9. 10. The IV spike (310) of Claim 1, wherein the spike portion comprises a bevel (124).

10. The IV spike (310) of Claim 1, wherein the at least one spike flow port is disposed adjacent to the spike portion.

11. The IV spike (310) of Claim 1, wherein the at least one spike flow port comprises a plurality of flow ports.

12. A method to transfer fluid from an IV container to a drip chamber, the method comprising:
engaging an engagement feature of an IV spike (310) according to claim 1 against a membrane of the IV container, wherein the membrane of the IV container is axially disposed between the upper axial retention feature (344) and the lower axial retention feature (346); and
directing flow from the IV container to the drip chamber through the IV spike (310).

13. The method of Claim 12, further comprising:
piercing the IV container by advancing the IV spike (310) through the membrane of the IV container; and
advancing the IV spike (310) through the IV container.

## Patentansprüche

1. IV-Spike (310), umfassend:
einen Spike-Körper, der aus einem ersten Material besteht und einen zu einer Spitze zulaufenden Spike-Abschnitt aufweist;
mindestens eine durch den Spike-Körper hindurch ausgebildete Durchflussöffnung;
eine untere Durchflussöffnung, die in Fluidverbindung mit der mindestens einen Durchflussöffnung steht;
und **gekennzeichnet durch**:
ein aus einem zweiten Material umspritztes Eingriffselement, das sich radial vom Spike-Körper weg erstreckt, wobei das Eingriffselement so ausgebildet ist, dass es den IV-Spike (310) in einem IV-Behälter hält, wobei das Eingriffselement umfasst:
ein oberes axiales Halteelement (344) mit einem oberen axial distalen Radius (344a) und einem oberen axial proximalen Radius (344b), wobei sich das obere axiale Halteelement (344) vom kleineren axial distalen Radius zum größeren oberen axial proximalen Radius (344b) verjüngt; und
ein unteres axiales Halteelement (346) mit einem unteren, axial distalen Radius (346a) und einem unteren, axial proximalen Radius (346b), wobei sich das untere axiale Halteelement (346) vom größeren, axial distalen Radius zum kleineren, unteren, axial proximalen Radius (346b) verjüngt.

2. IV-Spike (310) nach Anspruch 1, wobei das zweite Material ein nachgiebiges Material ist.

3. IV-Spike (310) nach Anspruch 1, wobei das erste Material Acrylnitril-Butadien-Styrol-Kunststoff umfasst.

4. IV-Spike (310) nach Anspruch 1, wobei das zweite Material einen höheren Reibungskoeffizienten aufweist als das erste Material.

5. IV-Spike (310) nach Anspruch 1, wobei das zweite Material ein thermoplastisches Elastomer oder Silikon umfasst.

6. IV-Spike (310) nach Anspruch 1, wobei sich das Eingriffselement entlang einer axialen Länge des Spike-Körpers erstreckt.

7. IV-Spike (310) nach Anspruch 1, wobei das Eingriffselement einen zylindrischen Körper umfasst.

8. IV-Spike (310) nach Anspruch 7, wobei das Eingriffselement in Umfangsrichtung um den Spike-Körper herum angeordnet ist.

9. IV-Spike (310) nach Anspruch 1, wobei der Spike-Abschnitt eine Abschrägung (124) aufweist.

10. IV-Spike (310) nach Anspruch 1, wobei die mindestens eine Durchflussöffnung des Spikes benachbart zum Spike-Abschnitt angeordnet ist.

11. IV-Spike (310) nach Anspruch 1, wobei die mindestens eine Durchflussöffnung des Spikes eine Vielzahl von Durchflussöffnungen umfasst.

12. Verfahren zum Übertragen von Flüssigkeit aus einem IV-Behälter in eine Tropfkammer, wobei das Verfahren umfasst:
Anlegen eines Eingriffselements eines IV-Spikes (310) gemäß Anspruch 1 an eine Membran des IV-Behälters, wobei die Membran des IV-Behälters axial zwischen dem oberen axialen Halteelement (344) und dem unteren axialen Halteelement (346) angeordnet ist; und
Leiten eines Flusses aus dem IV-Behälter durch den IV-Spike (310) in die Tropfkammer.

13. Verfahren nach Anspruch 12, das ferner umfasst:
Durchstechen des Infusionsbehälters durch Vorschieben des Infusionsspikes (310) durch die Membran des Infusionsbehälters; und
Vorschieben des Infusionsspikes (310) durch den Infusionsbehälter.

## Revendications

1. Pointe IV (310), comprenant :
un corps de pointe comprenant un premier matériau, et une section de pointe convergeant vers une extrémité ;
au moins un orifice d'écoulement de pointe formé à travers le corps de pointe ;
un orifice d'écoulement inférieur en communication fluidique avec ledit au moins un orifice d'écoulement de pointe ;
et **caractérisée par** :
un élément d'engagement appliqué par moulage par injection à partir d'un deuxième matériau et s'étendant radialement à l'écart du corps de pointe, l'élément d'engagement étant configuré pour retenir la pointe IV (310) à l'intérieur d'un récipient IV, l'élément d'engagement comprenant :
un élément de retenue axial supérieur (344) présentant un rayon axialement distal supérieur (344a) et un rayon axialement proximal supérieur (344b), l'élément de retenue axial supérieur (344) s'amincissant du plus petit rayon axialement distal au plus grand rayon axialement proximal supérieur (344b) ; et
un élément de retenue axial inférieur (346) présentant un rayon axialement distal inférieur (346a) et un rayon axialement proximal inférieur (346b), l'élément de retenue axial inférieur (346) s'amincissant du plus grand rayon axialement distal au plus petit rayon axialement proximal inférieur (346b).

2. La pointe IV (310) selon la revendication 1, dans laquelle le deuxième matériau est un matériau souple.

3. La pointe IV (310) selon la revendication 1, dans laquelle le premier matériau comprend un plastique d'acrylonitrile-butadiène-styrène.

4. La pointe IV (310) selon la revendication 1, dans laquelle le deuxième matériau présente un coefficient de frottement supérieur à celui du premier matériau.

5. La pointe IV (310) selon la revendication 1, dans laquelle le deuxième matériau comprend un élastomère thermoplastique ou du silicone.

6. La pointe IV (310) selon la revendication 1, dans laquelle l'élément d'engagement s'étend sur une longueur axiale du corps de pointe.

7. La pointe IV (310) selon la revendication 1, dans laquelle l'élément d'engagement comprend un corps cylindrique.

8. La pointe IV (310) selon la revendication 7, dans laquelle l'élément d'engagement est disposé circonférentiellement autour du corps de pointe.

9. La pointe IV (310) selon la revendication 1, dans laquelle la section de pointe comprend un biseau (124).

10. La pointe IV (310) selon la revendication 1, dans laquelle ledit au moins un orifice d'écoulement de pointe est disposé adjacent à la section de pointe.

11. La pointe IV (310) selon la revendication 1, dans laquelle ledit au moins un orifice d'écoulement de pointe comprend une pluralité d'orifices d'écoulement.

12. Procédé de transfert d'un fluide d'un récipient IV vers une chambre compte-gouttes, le procédé comprenant :
mettre en prise un élément d'engagement d'une pointe IV (310) selon la revendication 1 avec une membrane du récipient IV,
la membrane du récipient IV étant disposée axialement entre l'élément de retenue axial supérieur (344) et l'élément de retenue axial inférieur (346) ; et
diriger un écoulement du récipient IV vers la chambre compte-gouttes à travers la pointe IV (310).

13. Le procédé selon la revendication 12, comprenant en outre :
percer le récipient IV en faisant avancer la pointe IV (310) à travers la membrane du récipient IV ; et
faire avancer la pointe IV (310) à travers le récipient IV.
